(19) Europäisches Patentamt / European Patent Office / Office européen des brevets

(11) **EP 1 951 243 B1**

(12) **EUROPEAN PATENT SPECIFICATION**

(45) Date of publication and mention
of the grant of the patent:
**04.08.2010 Bulletin 2010/31**

(51) Int Cl.:
***A61K 31/506*** *(2006.01)*

(21) Application number: **06754477.5**

(86) International application number:
**PCT/EP2006/005960**

(22) Date of filing: **21.06.2006**

(87) International publication number:
**WO 2006/136391 (28.12.2006 Gazette 2006/52)**

(54) **TREATMENT OF SOLID TUMOR DISEASES WITH COMBINATIONS COMPRISING IMATINIB AND AN EFFLUX PUMP INHIBITOR**

VERFAHREN ZUR BEHANDLUNG VON SOLIDEN TUMOREN EINSCHLIESSLICH DER VERABREICHUNG EINER KOMBINATION MIT IMATINIB UND EINEM EFFLUXPUMPENHEMMER

TRAITEMENT DE TUMEURS SOLIDES AVEC DES COMBINAISONS COMPRENANT L'IMATINIB ET UN INHIBITEUR DE POMPE À EFFLUX

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 GB 0512843**
**03.02.2006 GB 0602264**

(43) Date of publication of application:
**06.08.2008 Bulletin 2008/32**

(73) Proprietor: **Novartis AG**
**4056 Basel (CH)**

(72) Inventors:
• **BIHOREL, Sebastien**
**F-93270 Sevran (FR)**
• **GROSS, Gerhard**
**79539 Lörrach (DE)**
• **LEMAIRE, Michel**
**F-68220 Buschwiller (FR)**
• **CAMENISCH, Gian, P.**
**CH-4125 Riehen (CH)**

(74) Representative: **Krauss, Jan**
**Forrester & Boehmert**
**Pettenkoferstrasse 20-22**
**80336 München (DE)**

(56) References cited:
**WO-A-2004/032925       WO-A2-2004/103374**
**WO-A2-2006/012958       US-A1- 2004 102 453**

• **BREEDVELD PAULINE ET AL: "The effect of Bcrp1 (Abcg2) on the in vivo pharmacokinetics and brain penetration of imatinib mesylate (Gleevec): implications for the use of breast cancer resistance protein and P-glycoprotein inhibitors to enable the brain penetration of imatinib in patients." CANCER RESEARCH. 1 APR 2005, vol. 65, no. 7, 1 April 2005 (2005-04-01), pages 2577-2582, XP007901255 ISSN: 0008-5472**
• **MAHON FRANCOIS-XAVIER ET AL: "MDR1 gene overexpression confers resistance to imatinib mesylate in leukemia cell line models." BLOOD, vol. 101, no. 6, 15 March 2003 (2003-03-15), pages 2368-2373, XP002404659 ISSN: 0006-4971**
• **THOMAS J ET AL: "Active transport of imatinib into and out of cells: Implications for drug resistance" BLOOD 01 DEC 2004 UNITED STATES, vol. 104, no. 12, 1 December 2004 (2004-12-01), pages 3739-3745, XP002404660 ISSN: 0006-4971**
• **DAI HAIQING ET AL: "Distribution of STI-571 to the brain is limited by P-glycoprotein-mediated efflux." THE JOURNAL OF PHARMACOLOGY AND EXPERIMENTAL THERAPEUTICS. MAR 2003, vol. 304, no. 3, March 2003 (2003-03), pages 1085-1092, XP002404662 ISSN: 0022-3565**
• **HAMADA AKINOBU ET AL: "Interaction of imatinib mesilate with human P-glycoprotein." THE JOURNAL OF PHARMACOLOGY AND EXPERIMENTAL THERAPEUTICS. NOV 2003, vol. 307, no. 2, November 2003 (2003-11), pages 824-828, XP007901256 ISSN: 0022-3565**

- KANO Y ET AL: "IN VITRO CYTOTOXIC EFFECTS OF A TYROSINE KINASE INHIBITOR STI571 IN COMBINATION WITH COMMONLY USED ANTILEUKEMIC AGENTS" BLOOD, W.B.SAUNDERS COMPANY, ORLANDO, FL, US, vol. 97, no. 7, 1 April 2001 (2001-04-01), pages 1999-2007, XP001035243 ISSN: 0006-4971
- LONARDI S ET AL: "Adjuvant chemotherapy in the treatment of high grade gliomas" CANCER TREATMENT REVIEWS, SAUNDERS, US, vol. 31, no. 2, April 2005 (2005-04), pages 79-89, XP004852579 ISSN: 0305-7372
- ABDOLLAHI ET AL: "Trimodal Therapy of U87 Human Glioblastoma Tumors in Mice Using Combined Radiotherapy, Temozolomide and Imatinib/Gleevec" INTERNATIONAL JOURNAL OF RADIATION: ONCOLOGY BIOLOGY PHYSICS, PERGAMON PRESS, US, vol. 63, 1 October 2005 (2005-10-01), page S259, XP005083448 ISSN: 0360-3016
- SCHINKEL ALFRED H ET AL: "Mammalian drug efflux transporters of the ATP binding cassette (ABC) family: An overview." ADVANCED DRUG DELIVERY REVIEWS, vol. 55, no. 1, 21 January 2003 (2003-01-21), pages 3-29, XP007901356 ISSN: 0169-409X

**Description**

The invention relates to treating a warm-blooded animal, especially a human, having a solid tumor disease comprising the use of a combination which comprises (a) N-{5-[4-(4-methyl-piperazino-methyl)-benzoylamido]-2-methylphenyl}-4-(3-pyridyl)-2-pyrimidine-amine as only active ingredient inhibiting the BCRP efflux pump, (b) at least one compound inhibiting the efflux pump active at the blood brain barrier selected from MDR1 and MRP2 selected from the group consisting of: (1) [3'-desoxy-3'-oxo-MeBmt][1]-Ciclosporin, (2) [3'-desoxy-3'-oxo-MeBmt][1]-[Val][2]-Ciclosporin, (3) [3'-desoxy-3'-oxo-MeBmt][1]-[Nva][2]-Ciclosporin, (4) Cyclo-[Pec-MeVal-Val-MeAsp(β-P-t-Bu)-Melle-Melle-Gly-MeVal-Tyr(Me)-L-Lact], (5) Cyclo-[Pec-MeVal-Val-MeAsp-Melle-Melle-Gly-MeVal-Tyr(Me)-D-Lact], and (6) Verapamil, (c) optionally an antineoplastic agent, especially as defined herein, (d) optionally an alkylating agent and (e) optionally, hydroxyurea for the treatment of a solid tumor disease.

The mesylate salt of N-{5-[4-(4-methyl-piperazino-methyl)-benzoylamido]-2-methylphenyl}-4-(3-pyridyl)-2-pyrimidine-amine (Imatinib mesylate, STI571) is marketed under the brand Glivec® (Gleevec®). Glivecc® is a tyrosine kinase inhibitor suitable for the treatment of chronic myeloid leukemia and GIST (gastro-intestinal stromal tumors). New clinical studies in glioblastoma multiform (GBM) patients show response in terms of tumor shrinking and progression free survival when Glivec® is administered together with hydroxyurea (G. Dresemann, ASCO 2004, Abstract 1550; Reardon et al, J Clin Oncol. 2005 Dec 20, 23(36): 9359-68; Dresemann, Ann Oncol. 2005 Oct,16(10):1702-8). Since brain uptake of ST1571 is rather limited, as shown in animal studies, this limits also the pharmacological effect of Glivec®. It was suggested to improve the brain penetration of imatinib mesylate by coadministering a BCRP inhibitor and, optionally, a P-gp inhibitor ("The effect of Bcrpi (Abcg2) on the in vivo pharmacokinetics and brain penetration of imatinib mesylate (Gleevec): implications for the use of BCRP and P-gp inhibitors to enable the barin penetration of imatinib in patients." Breedveld P, Puim D, Cipriani G, Wielinga P, van Tellingen O, Schinkel AH et al. Cancer Res. 2005, Apr 1 ;65(7): 2577-82). Breedveld P. et al describes the effect of P-gp and Bcrp1 inhibitors on the brain penetration of intravenous imatinib in mice. The authors show that the inhibitor elacridar significantly increases the brain penetration of imatinib (see Figure 4a).

Attention is brought to the fact that elacridar is both an inhibitor of Bcrp1 and P-gp, as mentioned by the authors themselves on page 2581, left side, last sentence of the third paragraph. Thus it remains unclear if the shown effect of elacridar is due to P-gp or Bcrp1 inhibition. The authors concluded that the positive effect of the drug was due to inhibition of Bcrp1 and not of P-gp. Thus, the skilled artisan when reading D1 would likely avoid the use of a P-gp inhibitor alone in the combination with imatinib, but focus on Bcrp1 inhibitors, or at least on the combination of a Bcrp1 and a P-gp inhibitor with imatinib.

WO 2006/012958 teaches the use of a combination comprising a) a BCRP (Breast Cancer Resistance Protein) inhibitor, b) imatinib and c) a P-gp (P-Glycoprotein) inhibitor and imatinib and a further BCRP inhibitor.

Mahon, F-X. et al. Blood, vol. 101, 2003, no. 6, page 2368-2373 analyses the effect of imatinib on cell viability in a P-gp overexpressing CML-cell line (K562/DOX) which was treated with verapamil or PSC833 (valspodar). The paper differs from the teaching of present application in that the authors look only at the toxic effect of imatinib on CML derived cell lines in an *in-vitro* model wherein P-gp is artificially overexpressed.

Dai H. et al., J. Pharm. Exp. Ther., vol. 304, 2003, no. 3, pages 1085-1092 refers to the question if the drug imatinib is a target of the P-gp mediated efflux in the blood-brain barrier. Mostly, the paper examines the role of P-gp in a knock-out mouse model, wherein the blood-brain distribution of imatinib after drug administration is analysed. The presently claimed combinations show to have a surprisingly better effect on the brain/plasma distribution of imatinib than the P-gp inhibitor LY335979.

N-{5-[4-(Piperazino-methyl)-benzoylamido]-2-methylphenyl}-4-(3-pyridyl)-2-pyrimidine-amine (CGP74588) is an active metabolite of Imatinib.

Surprisingly, it was now found that co-administration of STI571 with at least one compound inhibiting an efflux pump active at the blood brain barrier selected from MDR1 and MRP2 selected from the group consisting of (1) [3'-desoxy-3'-oxo-MeBmt]'-Ciclosporin, (2) [3'-desoxy-3'-oxo-MeBmt][1]-[Val][2]-Ciclosporin, (3) [3'-desoxy-3'-oxo-MeBmt][1]-[Nva][2]-Ciclosporin, (4) Cyclo-[Pec-MeVal-Val-MeAsp(β-P-t-Bu)-Melle-Melle-Gly-MeVal-Tyr(Me)-L-Lact], (5) Cyclo-[Pec-MeVal-Val-MeAsp-Melle-Melle-Gly-MeVal-Tyr(Me)-D-Lact], and (6) Verapamil, represents a route to significantly improve ST1571 brain penetration also in the absence of any active ingredient inhibiting BCRP other than STI571 itself. Hence, the present invention relates in a first aspect to the use of a combination comprising (a) N-{5-[4-(4-methyl-piperazino-methyl)-benzoylamido]-2-methylphenyl}-4-(3-pyridyl)-2-pyrimidine-amine, as the only active ingredient inhibiting the BCRP efflux pump, (b) at least one compound inhibiting an efflux pump active selected from MDR1 and MRP2 selected from the group consisting of (1) [3'-desoxy-3'-oxo-MeBmt][1]-Ciclosporin, (2) [3'-desoxy-3'-oxo-MeBmt][1]-[Val][2]-Ciclosporin, (3) [3'-desoxy-3'-oxo-MeBmt][1]-[Nva][2]-Ciclosporin, (4) Cyclo-[Pec-MeVal-Val-MeAsp(β-P-t-Bu)-Melle-Melle-Gly-MeVal-Tyr(Me)-L-Lact], (5) Cyclo-[Pec-MeVal-Val-MeAsp-Melle-Melle-Gly-MeVal-Tyr(Me)-D-Lact], and (6) Verapamil, (c) optionally an antineoplastic agent, especially as defined herein, and (d) optionally an alkylating agent and (e) optionally, hydroxyurea, in which the compounds can also be present in the form of their pharmaceutically acceptable salts or any hydrate thereof, and optionally at least one pharmaceutically acceptable carrier; for simultaneous, separate or sequential use for the preparation of a medicament for the treatment of a solid tumor disease.

The efflux pumps MDR1 and MRP2 are known to be active at the blood brain barrier. Hence, the present invention is in particular useful for the treatment of a solid tumor diseases located in the central nervous system (CNS), especially glioma, such as glioblastoma multiforme. As used herein, the expression "glioma" includes all primary intrinsic neoplasms of the brain and spinal cord, e.g. astrocytomas, ependymomas, neurocytomas or meningiomas. In a broader aspect, the combinations as described herein can be used for any solid tumor disease by preventing efflux of the N-{5-[4-(4-methyl-piperazino-methyl)-benzoylamido]-2-methylphenyl}-4-(3-pyridyl)-2-pyrimidine-amine from the tumor cells.

The term "solid tumor" as used herein especially means breast cancer, cancer of the colon and generally the GI tract, lung cancer, in particular small-cell lung cancer, and non-small-cell lung cancer, head and neck cancer, genitourinary cancer, e.g. cervical, uterine, ovarian, testicles, prostate or bladder cancer; Hodgkin's disease or Kaposi's sarcoma. Depending on the tumor type and the particular combination used a decrease of the tumor volume can be obtained.

The combinations of the present invention can be used in the form of a combined preparation or a pharmaceutical composition.

The term "a combined preparation", as used herein defines especially a "kit of parts" in the sense that the combination partners (a), (b), (c), (d) and (e) as defined above can be dosed independently or by use of different fixed combinations with distinguished amounts of the combination partners (a), (b), (c), (d) and (e), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners. The ratio of the total amounts of the combination partner (a), (b), (c), (d) and (e) to each other being administered in the combined preparation can be varied, e.g. in order to cope with the needs of a patient sub- population to be treated or the needs of the single patient which different needs can be due to the particular disease, age, sex, body weight, etc. of the patients. Preferably, there is at least one beneficial effect, e.g., a mutual enhancing of the effect of the combination partners (a), (b), (c), (d) and (e), in particular a synergism, e.g. a more than additive effect, additional advantageous effects, less side effects, a combined therapeutic effect in a non-effective dosage of one or both of the combination partners (a), (b), (c), (d) and (e), and very preferably a strong synergism of the combination partners ((a), (b), (c), (d) and (e).

The term "treatment" includes the treatment of a patient in need thereof resulting in a delay of progression of the solid tumor disease. The term "delay of progression" as used herein means administration of the combination to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

It will be understood that references to the combination partners are meant to also include the pharmaceutically acceptable salts or pro-drugs. If the combination partners have, for example, at least one basic center, they can form acid addition salts. Corresponding acid addition salts can also be formed having, if desired, an additionally present basic center. A combination partners (a), (b), (c) or (d) having an acid group (for example COOH) can also form salts with bases. N-{5-[4-(4-methyl-piperazino-methyl)-benzoylamido]-2-methylphenyl}-4-(3-pyridyl)-2-pyrimidine-amine, i.e. combination partner (a), is preferably used in the present invention in the form of its monomesylate salt (STI571).

N-phenyl-2-pyrimidine-amine derivatives are especially disclosed in US 5,521,184. N-{5-[4-(4-methyl-piperazino-methyl)-benzoylamido]-2-methylphenyl}-4-(3-pyridyl)-2-pyrimidine-amine can be prepared as disclosed in US 5,521,184. The monomesylate salt of N-{5-[4-(4-methyl-piperazino-methyl)-benzoylamido]-2-methylphenyl}-4-(3-pyridyl)-2-pyrimidine-amine can be prepared and formulated, e.g., as described in Examples 4 and 6 of WO 99/03854 or as described in WO03/090720. N-{5-[4-(Piperazino-methyl)-benzoylamido]-2-methylphenyl}-4-(3-pyridyl)-2-pyrimidine-amine (CGP74588) can be prepared as described in US 2004-0102453.

A compound inhibiting the efflux pump MDR1 (P-gp inhibitor) is selected from verapamil, [3'-desoxy-3'-oxo-MeBmt][1]-Ciclosporin, [3'-desoxy-3'-oxo-MeBmt][1]-[Val][2]-Ciclosporin and [3'-desoxy-3'-oxo-MeBmt][1][Nva][2]-Ciclosporin, which Ciclosporins are disclosed in EP 0 296 122 in Example H as cyclosporins 1.37, 1.38 and 1.39, respectively, as well as Cyclo-[Pec-MeVal-Val-MeAsp([beta]-P-t-Bu)-MeIle-MeIle-Gly-MeVal-Tyr(Me)-L-Lact] and Cyclo-[Pec-MeVal-Val-MeAsp-MeIle-MeIle-Gly-MeVal-Tyr(Me)-D-Lact], disclosed in EP 0 360 760 as Examples 52 and 1 (first compound), respectively. With regard to all aspects of the present invention, preferably [3'-desoxy-3'-oxo-MeBmt][1]-[Val][2]-Ciclosporin A, also known as valspodar, hereinafter also referred to as PSC833, known from EP 0 296 122 (Example H) is used as the P-gp inhibitor. PSC833 can be administered in the form of the galenical composition disclosed in WO 93/20833.

The term "alkylating agents" as used herein includes, but is not limited to cyclophosphamide, ifosfamide and melphalan. Cyclophosphamide can be administered, e.g., in the form as it is marketed, e.g. under the trademark CYCLOSTIN™. Ifosfamide can be administered, e.g., in the form as it is marketed, e.g. under the trademark HOLOXAN™.

One preferred alkylating agent is iemozoiomide.

The term "antineoplastic agents" as used herein includes, but is not limited to aromatase inhibitors, antiestrogens, topoisomerase I inhibitors, topoisomerase II inhibitors, microtubule active agents, antineoplastic antimetabolites, platin compounds, compounds decreasing the protein kinase activity and further anti-angiogenic compounds, gonadorelin agonists, anti-androgens, bisphosphonates and trastuzumab.

The term "aromatase inhibitors" as used herein relates to compounds which inhibit the estrogen production, i.e. the conversion of the substrates androstenedione and testosterone to estrone and estradiol, respectively. The term includes, but is not limited to steroids, especially exemestane and formestane and, in particular, non-steroids, especially aminoglutethimide, vorozole, fadrozole, anastrozole and, very especially, letrozole. Exemestane can be administered, e.g., in the form as it is marketed, e.g. under the trademark AROMASIN™. Formestane can be administered, e.g., in the form as it is marketed, e.g. under the trademark LENTARON™. Fadrozole can be administered, e.g., in the form as it is marketed, e.g. under the trademark AFEMA™. Anastrozole can be administered, e.g., in the form as it is marketed, e.g. under the trademark ARIMIDEX™. Letrozole can be administered, e.g., in the form as it is marketed, e.g. under the trademark FEMARA™ or FEMAR™. Letrozole has been specifically described in the European patent No. 0 236 940 published on September 16, 1987, as well as in US patent No. 4,978,672 published on December 18, 1990, and Japanese Patent No. 2018112 all in the name of the applicant. Aminoglutethimide can be administered, e.g., in the form as it is marketed, e.g. under the trademark ORIMETEN™.

The term "antiestrogens" as used herein relates to compounds which antagonize the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX™. Raloxifene hydrochloride can be administered, e.g., in the form as it is marketed, e.g. under the trademark EVIST A™. Fulvestrant can be formulated as disclosed in US 4,659,516 or it can be administered, e.g., in the form as it is marketed, e.g. under the trademark FASLODEX™.

The term "topoisomerase I inhibitors" as used herein includes, but is not limited to topotecan, irinotecan, 9-nitrocamptothecin and the macromolecular camptothecin conjugate PNU-166148 (compound A1 in WO99/17804). Irinotecan can be administered, e.g., in the form as it is marketed, e.g. under the trademark CAMPTOSAR™. Topotecan can be administered, e.g., in the form as it is marketed, e.g. under the trademark HYCAMTIN™.

The term "topoisomerase II inhibitors" as used herein includes, but is not limited to the antra-cyclines doxorubicin (including liposomal formulation, e.g. CALYX™), epirubicin, idarubicin and nemorubicin, the anthraquinones mitoxantrone and losoxantrone, and the podophillotoxines etoposide and teniposide. Etoposide can be administered, e.g., in the form as it is marketed, e.g. under the trademark ETOPOPHOS™. Teniposide can be administered, e.g., in the form as it is marketed, e.g. under the trademark VM 26-BRISTOL™. Doxorubicin can be administered, e.g., in the form as it is marketed, e.g. under the trademark ADRIBLASTIN™. Epirubicin can be administered, e.g., in the form as it is marketed, e.g. under the trademark FARMORUBICIN™. Idarubicin can be administered, e.g., in the form as it is marketed, e.g. under the trademark ZAVEDOS™. Mitoxantrone can be administered, e.g., in the form as it is marketed, e.g. under the trademark NOVANTRON™.

The term "microtubule active agents" relates to microtubule stabilizing and microtubule destabilizing agents including, but not limited to the taxanes paclitaxel and docetaxel, the vinca alkaloids, e.g., vinblastine, especially vinblastine sulfate, vincristine especially vincristine sulfate, and vinorelbine and epothilones. Paclitaxel (Taxol® is a natural product with antitumor activity. TAXOL is obtained via a semi-synthetic process from Taxus baccata . The chemical name for paclitaxel is 5(beta),20-Epoxy-1,2(alpha),4,7(beta),10(beta),13(alpha)-hexahydroxytax-11-en-9-one 4,10-diacetate 2-benzoate 13-ester with (2 R,3 S )-N-benzoyl-3-phenylisoserine. Docetaxel can be administered, e.g., in the form as it is marketed, e.g. under the trademark TAXOTERE™. Vinblastine sulfate can be administered, e.g., in the form as it is marketed, e.g. under the trademark VINBLASTIN R.P.™. Vincristine sulfate can be administered, e.g., in the form as it is marketed, e.g. under the trademark FARMISTIN™.

The term "antineoplastic antimetabolites" includes, but is not limited to 5-fluorouracil, capecitabine, gemcitabine, methotrexate and edatrexate. Capecitabine can be administered, e.g., in the form as it is marketed, e.g. under the trademark XELODA™. Gemcitabine can be administered, e.g., in the form as it is marketed, e.g. under the trademark GEMZAR™. The term "platin compounds" as used herein includes, but is not limited to carboplatin, cis- platin and oxaliplatin. Carboplatin can be administered, e.g., in the form as it is marketed, e.g. under the trademark CARBOPLAT™. Oxaliplatin can be administered, e.g., in the form as it is marketed, e.g. under the trademark ELOXATIN™.

The term "compounds decreasing the protein kinase activity and further anti-angiogenic compounds" as used herein includes, but is not limited to compounds decreasing the activity of the epidermal growth factor (EGF) of the epidermal growth factor (EGF), the vascular endothelial growth factor (VEGF), the platelet derived growth factor (PDGF) and/or the protein kinase C and anti-angiogenic compounds having another mechanism for their activity.

Compounds which decreases the activity of VEGF are especially compounds which inhibit the VEGF receptor tyrosine kinase, compounds which inhibit a VEGF receptor and compounds binding to VEGF, and are in particular those compounds, proteins and monoclonal antibodies generically and specifically disclosed in WO 98/35958, WO 00/09495, WO 00/27820, WO 00/59509, WO 98/11223, WO 00/27819 and EP 0 769 947; those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58, 1998, 3209-3214, and by J. Mordenti et al in Toxicologic Pathology, Vol. 27, no. 1 , pp 14-21, 1999; in WO 00/37502 and WO 94/10202; Angiostatin™, described by M. S. O'Reilly et al, Cell 79, 1994, 315-328; and Endostatin™, described by M. S. O'Reilly et al, Cell 88, 1997, 277-285;
compounds which decrease the activity of the epidermal growth factor (EGF) are especially compounds which inhibit the EGF receptor tyrosine kinase, compounds which inhibit the EGF receptor and compounds binding to EGF, and are in particular those compounds generically and specifically disclosed in WO 97/02266, EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/33980;
compounds which decreases the activity of the protein kinase C are especially those stauro-sporine derivatives disclosed in EP 0 296 110 (pharmaceutical preparation described in WO 00/48571) which compounds are protein kinase C inhibitors; in each case where citations of patent applications or scientific publications are given in particular in the compound claims and the final products of the working examples, the subject-matter of the final products. The pharmaceutical preparations and the claims of such patent publications is hereby incorporated into the present application by reference to this publications. Comprised are likewise the corresponding stereoisomers as well as the corresponding crystal modifications, e.g. solvates and polymorphs, which are disclosed therein. The compounds used as active ingredients in the combinations disclosed herein can be prepared and administered as described in the cited documents, respectively.

Further anti-angiogenic compounds are thalidomide (THALOMID), SU5416, and celecoxib (Celebrex);

The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX™. Abarelix can be formulated, eg. as disclosed in US 5,843,901.

The term "anti-androgens" as used herein includes, but is not limited to bicalutamide (CASODEX™), which can be formulated, e.g. as disclosed in US 4,636,505.

The term "bisphosphonates" as used herein includes, but is not limited to etridonic acid, clodronic acid, tiludronic acid, pamidronic acid, alendronic acid, ibandronic acid, risedronic acid and zoledronic acid. "Etridonic acid" can be administered, e.g., in the form as it is marketed, e.g. under the trademark DIDRONEL™. "Clodronic acid" can be administered, e.g., in the form as it is marketed, e.g. under the trademark BONEFOS™. "Tiludronic acid" can be administered, e.g., in the form as it is marketed, e.g. under the trademark SKELID™. "Pamidronic acid" can be administered, e.g., in the form as it is marketed, e.g. under the trademark AREDIA™. "Alendronic acid" can be administered, e.g., in the form as it is marketed, e.g. under the trademark FOSAMAX™. "Ibandronic acid" can be administered, e.g., in the form as it is marketed, e.g. under the trademark BONDRANAT™. "Risedronic acid" can be administered, e.g., in the form as it is marketed, e.g. under the trademark ACTONEL™. "Zoledronic acid" can be administered, e.g., in the form as it is marketed, e.g. under the trademark ZOMETA™. Zoledronic acid has been specifically described in the European patent No. 0 275 821, published on July 27, 1988, as well as in US patent No. 4,939,130 granted on July 03, 1990, and Japanese Patent No. 2744238 all in the name of the applicant. The term "Zoledronic acid" also includes pharmacologically acceptable salt thereof or any hydrate thereof such as anhydrous zoledronic acid, zoledronic acid monohydrate, disodium zeldronate, trisodium zeldronate.

"Trastuzumab" can be administered, e.g., in the form as it is marketed, e.g. under the trademark HERCEPTIN™).

The structure of the active agents identified by code nos., generic or trade names may be taken from the actual edition of the standard compendium "The Merck Index" or from databases, e.g. Patents International (e.g. IMS World Publications).

A combination which comprises (a) N- {5-[4-(4-methyl-piperazino-methyl)-benzoylamido]-2-methylphenyl}-4-(3-pyridyl)-2-pyrimidine- amine, as the only active ingredient inhibiting the BCRP efflux pump, (b) at least one compound inhibiting an efflux pump active selected from MDR1 and MRP2 selected from the group consisting of (1) [3'-desoxy-3'-oxo-MeBmt][1]-Ciclosporin, (2) [3'-desoxy-3'-oxo-MeBmt][1]-[Val][2]-Ciclosporin, (3) [3'-desoxy-3'-oxo-MeBmt][1]-[Nva][2]-Ciclosporin, (4) Cyclo-[Pec-MeVal-Val-MeAsp(β-P-t-Bu)-Melle-Melle-Gly-MeVal-Tyr(Me)-L-Lact], (5) Cyclo-[Pec-Me-Val-Val-MeAsp-Melle-Melle-Gly-MeVal-Tyr(Me)-D-Lact], and (6) Verapamil, (c) optionally an antineoplastic agent, especially as defined herein, and (d) optionally an alkylating agent and (e) optionally, hydroxyurea, in which the compounds can also be present in the form of their pharmaceutically acceptable salts-or any hydrate thereof, and optionally at least one pharmaceutically acceptable carrier; for simultaneous, separate or sequential use, will be referred to hereinafter as a COMBINATION OF THE INVENTION.

It can be shown by established test models and in particular those test models described herein that a COMBINATION OF THE INVENTION results in a significant improvement of up-take of STI571 into solid tumor cells and STI571 brain penetration. The improved up-take and penetration results in a more efficacious treatment of solid tumor diseases, especially solid tumor diseases of the central nervous system. This advantage results in the further benefit that for the same clinical effect lower doses of the active ingredients of the COMBINATION OF THE INVENTION can be used, for example, that the dosages need not only often be smaller but also can be applied less frequently thus diminishing the incidence of side-effects. This is in accordance with the desires and requirements of the patients to be treated.

The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

The study in FVB mice described in the Examples below of *in situ* brain perfusion indicates that the transport of imatinib across the blood-brain barrier is limited by saturable processes $( K_M^{app}$ ~5-10 $\mu M)$. Experiments in FVB *mdr1a1b*(-/-) mice strongly suggest that P-glycoprotein and Breast-Cancer Resistance Protein are involved. In FVB wild-type mice, co-perfusion of valspodar 10 $\mu M$ with imatinib 0.5 $\mu M$ can markedly enhance the distribution of imatinib into the brain.

Suitable clinical studies are, for example, open label non-randomized, dose escalation studies in patients with glioblastoma multiforme. Such studies prove in particular the improved brain penetration of STI571 and therapeutic efficacy of a COMBINATION OF THE INVENTION. The beneficial effects on solid tumor diseases can be determined directly through the results of these studies or by changes in the study design which are known as such to a person skilled in the art.

The COMBINATION OF THE INVENTION can also be applied in combination with surgical intervention, mild prolonged whole body hyperthermia and/or irradiation therapy.

In one embodiment of the invention the chemotherapeutic agent is an antineoplastic agent selected from aromatase inhibitors, antiestrogens, topoisomerase I inhibitors, topoisomerase II inhibitors, microtubule active agents, antineoplastic antimetabolites, platin compounds, compounds decreasing the protein kinase activity and further anti-angiogenic compounds, gonadorelin agonists, anti-androgens, bisphosphonates and trastuzumab. In such embodiment the antineoplastic agent is especially selected from pamidronic acid, zoledronic acid and letrozole, preferably zoledronic acid and letrozole.

One preferred embodiment of the invention relates to the use of a COMBINATION OF THE INVENTION for the treatment of glioma. Preferably, in such embodiment the COMBINATION OF THE INVENTION comprises hydroxyurea.

It is one objective of this invention to provide a pharmaceutical composition comprising a quantity, which is jointly therapeutically effective against a solid tumor disease comprising the COMBINATION OF THE INVENTION. In this composition, the combination partners (a), (b), (c) and (d) can be administered together, one after the other or separately in one combined unit dosage form or in separate unit dosage forms. The unit dosage form may also be a fixed combination.

The pharmaceutical compositions for separate administration of the combination partners (a), (b), (c), (d) and (e) and for the administration in a fixed combination, i.e. a single galenical compositions comprising at least two combination partners, according to the invention can be prepared in a manner known per se and are those suitable for enteral, such as oral or rectal, and parenteral administration to mammals (warm-blooded animals), including humans, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar- coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the COMBINATION OF THE INVENTION may be administered simultaneously or sequentially and in any order, and the components may be administered separately or as a fixed combination. For example, the method of treatment of a solid tumor disease according to the invention may comprise (i) administration of the combination partner (a) in free or pharmaceutically acceptable salt form and (ii) administration of the combination partners (b), (c), (d) or (e) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert in vivo to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

N-{5-[4-(4-methyl-piperazino-methyl)-benzoylamido]-2-methylphenyl}-4-(3-pyridyl)-2-pyrimidine-amine monomesylate, is preferably administered to a human in a dosage in the range of about 25 to 1000 mg/day, more preferably 50 to 800 mg/day and most preferably 100 to 400 mg/day. Unless stated otherwise herein, the compound is preferably administered from one to four times per day.

Fadrozole may be administered orally to a human in a dosage range varying from about 0.5 to about 10 mg/day, preferably from about 1 to about 2.5 mg/day.

Letrozole may be administered orally to a human in a dosage range varying from about 0.1 to about 10 mg/day, preferably from about 0.5 to about 5 mg/day, most preferably from about 1 to about 2.5 mg/day. Examples of compositions containing letrozole are well known in the art, e.g. Physicians' Desk Reference Copyright © 2001 (Medical Economics Company Inc). Single doses of Zoledronic acid should preferably not exceed 8 mg; preferably 4 mg more preferably from about 0.5 to about 4 mg. Compositions containing zoledronic acid are described in the art, e.g. in the International patent application US01/14886 filed on May 5, 2001, and published on November 29, 2001.

Exemestane may be administered orally to a human in a dosage range varying from about 5 to about 200 mg/day, preferably from about 10 to about 25 mg/day, or parenterally from about 50 to 500 mg/day, preferably from about 100 to about 250 mg/day. If the drug shall be administered in a separate pharmaceutical composition, it can be administered in the form disclosed in GB 2,177,700.

Formestane may be administered parenterally to a human in a dosage range varying from about 100 to 500 mg/day, preferably from about 250 to about 300 mg/day.

Anastrozole may be administered orally to a human in a dosage range varying from about 0.25 to 20 mg/day, preferably from about 0.5 to about 2.5 mg/day.

Aminogluthemide may be administered to a human in a dosage range varying from about 200 to 500 mg/day. Tamoxifen citrate may be administered to a human in a dosage range varying from about 10 to 40 mg/day.

Vinblastine may be administered to a human in a dosage range varying from about 1.5 to 10 mg/m$^2$ day.

Vincristine sulfate may be administered parenterally to a human in a dosage range varying from about 0.025 to 0.05 mg/kg body weight * week.

Vinorelbine may be administered to a human in a dosage range varying from about 10 to 50 mg/m$^2$ day.

Etoposide phosphate may be administered to a human in a dosage range varying from about 25 to 115 mg/m$^2$ day, e.g. 56.8 or 113.6 mg/m$^2$ day.

Teniposide may be administered to a human in a dosage range varying from about 75 to 150 mg about every two weeks.

Doxorubicin may be administered to a human in a dosage range varying from about 10 to 100 mg/m$^2$ day, e.g. 25 or 50 mg/m$^2$ day.

Epirubicin may be administered to a human in a dosage range varying from about 10 to 200 mg/m$^2$ day. Idarubicin may be administered to a human in a dosage range varying from about 0.5 to 50 mg/m$^2$ day.

Mitoxantrone may be administered to a human in a dosage range varying from about 2.5 to 25 mg/m$^2$ day.

Paclitaxel may be administered to a human in a dosage range varying from about 50 to 300 mg/m$^2$ day.

Docetaxel may be administered to a human in a dosage range varying from about 25 to 100 mg/m$^2$ day.

Cyclophosphamide may be administered to a human in a dosage range varying from about 50 to 1500 mg/m$^2$ day.

Melphalan may be administered to a human in a dosage range varying from about 0.5 to 10 mg/m$^2$ day.

5-Fluorouracil may be administered to a human in a dosage range varying from about 50 to 1000 mg/m$^2$ day, e.g. 500 mg/m$^2$ day.

Capecitabine may be administered to a human in a dosage range varying from about 10 to 1000 mg/m$^2$ day.

Gemcitabine hydrochloride may be administered to a human in a dosage range varying from about 1000 mg/week.

Methotrexate may be administered to a human in a dosage range varying from about 5 to 500 mg/m$^2$ day.

Topotecan may be administered to a human in a dosage range varying from about 1 to 5 mg/m$^2$ day.

Irinotecan may be administered to a human in a dosage range varying from about 50 to 350 mg/m$^2$ day.

Carboplatin may be administered to a human in a dosage range varying from about 200 to 400 mg/m$^2$ about every four weeks.

Cisplatin may be administered to a human in a dosage range varying from about 25 to 75 mg/m$^2$ about every three weeks.

Oxaliplatin may be administered to a human in a dosage range varying from about 50 to 85 mg/m$^2$ every two weeks.

Alendronic acid may be administered to a human in a dosage range varying from about 5 to 10 mg/day

Clodronic acid may be administered to a human e.g. in a dosage range varying from about 750 to 1500 mg/day.

Etridonic acid may be administered to a human in a dosage range varying from about 200 to 400 mg/day. Ibandronic acid may be administered to a human in a dosage range varying from about 1 to 4 mg every three to four weeks.

Risedronic acid may be administered to a human in a dosage range varying from about 20 to 30 mg/day.

Pamidronic acid may be administered to a human in a dosage range varying from about 15 to 90 mg every three to four weeks.

Tiludronic acid may be administered to a human in a dosage range varying from about 200 to 400 mg/day.

Trastuzumab may be administered to a human in a dosage range varying from about 1 to 4 mg/m$^2$ week.

Bicalutamide may be administered to a human in a dosage range varying from about 25 to 50 mg/m$^2$ day.

Paclitaxel may be administered to a human in a dosage range varying from about 135 mg/m$^2$ to about 175 mg/m$^2$ administered intravenously every 3 weeks, or 100 mg/m$^2$ every 2 weeks. The succinate salt described in Example 62 of WO 98/35958 may be administered to a human in a dosage range of about 50 to 1500, more preferably about 100 to 750, and most preferably 250 to 500, mg/day.

Furthermore, the present invention pertains to the use of a COMBINATION OF THE INVENTION for the treatment of a solid tumor disease and for the preparation of a medicament for the treatment of a solid tumor disease.

Moreover, the present invention provides a commercial package comprising as active ingredients COMBINATION OF THE INVENTION, together with instructions for simultaneous, separate or sequential use thereof in the treatment of a solid tumor disease.

The following Example illustrates the invention described above, but is not, however, intended to limit the scope of the invention in any way. Other test models known as such to the person skilled in the pertinent art can also determine the beneficial effects of the

COMBINATION OF THE INVENTION.

Abbreviations

| | |
|---|---|
| AUC | Area under the concentration-time curve |
| AUC$_{last}$ | Area under the concentration-time curve from zero to the last measured time point |
| %CV | Coefficient of variation |
| HPLC | High performance liquid chromatography |
| Kp | Partition constant |
| LOQ | Limit of quantification |
| PEG | Polyethylene glycol |
| Rsq | Square of correlation coefficient (goodness of fit statistic) |
| SD | Standard deviation of the mean |
| T$_{1/2z}$ | Apparent elimination half-life |
| V$_{dss}$ | Volume of distribution at steady-state |

Example 1: *In situ* Brain Perfusion in Mice

Imatinib 0.5 $\mu$mol/L is administered in FVB mice using the in situ brain perfusion technique. After 1min perfusion, the brain uptake of the drug is low (net transport constant - 1 $\mu$L/g/s). When valspodar 25 $\mu$M is co-perfused, the brain uptake of imatinib is enhanced by a factor 2.5.

Example 2: *In vivo* Study in Mice

Imatinib 12.5 mg/kg is administered intravenously in FVB mice: 1 h post dose, the brain penetration of the drug is low (brain-to-plasma concentration ratios < 0.10). When valspodar 10 mg/kg is co-administered i.v., 1 h post dose, the brain penetration of imatinib is enhanced by a factor 4 to 5.

Example 3: *In vivo* Study in Rats

The blood and brain pharmacokinetics of imatinib can be studied in Hanover Wistar rats over 24 h. The drug (12.5 mg/kg) is administered intravenously with or without valspodar (10 mg/kg).

Group A: the rats receive successively [14C]STI571 12.5 mg-base/kg (~7.4 MBq/kg) in saline 0.9% by injection in one exposed saphenous vein (2 ml_/kg) and a mixture of ethanol - PEG 200 - glucose 5 % (2:6:2 v/v/v) by injection in the other one (2 mL/kg).

Group B: the rats receive successively [14C]STI571 12.5 mg-base/kg (~7.4 MBq/kg) in saline 0.9% by injection in one exposed saphenous vein (2 ml_/kg) and PSC833 (valspodar) 10 mg/kg in ethanol - PEG 200 - glucose 5 % (2:6:2 v/v/v) by injection in the other one (2 mL/kg).

Group C: the rats receive [14C]inulin 10 mg/kg (~1.9 MBq/kg; PerkinElmer, Boston, USA) in saline 0.9 % by injection in the exposed saphenous vein.

At 0.25, 0.5, 1 , 2, 4, 8 and 24 h after administration, rats in group A and B are sacrificed after anesthesia (n=3 per group), whereas mice in group C are sacrificed 0.08 h after administration (n=3). Blood, plasma, brain are collected for each animal.

All samples are analyzed for total radioactivity by liquid scintillation counting (LSC). In brief, 50 $\mu$L blood samples are successively mixed and incubated with 0.5 mL Soluene 350-isopropanol 1 :1 (v/v), 0.2 mL $H_2O_2$ 30 % (overnight), 0.5 mL HCl 2N (20 min) and 5 mL IrgaSafe (Packard) (overnight). 50 $\mu$L plasma samples are successively mixed and incubated with 0.5 mL Soluene 350-isopropanol 1:1 (v/v) (overnight), 0.5 ml. HCl 2N (20 min) and 5 mL IrgaSafe (overnight). 250 $\mu$L brain homogenate samples are treated by 2 mL Soluene 350 (overnight), 0.5 mL HCl 2N (20 min) and 17.5 mL Irgasafe (overnight). All samples are counted in Tricarb liquid scintillation analyzers (Packard). Automatic external standard techniques (quench compensation) are used to determine the efficiency of the respective radiometric analysis; observed data (cpm) are converted to disintegrations per minute (dpm).

In group A and B, the samples are also analyzed for unchanged STI571 by liquid chromatography method-based reverse isotope dilution (LC-RID). In brief, the concentrations of unchanged STI571 in blood (200 $\mu$L), plasma (200 $\mu$L) and brain (500 $\mu$L of homogenate) are determined by Liquid Chromatography - Reverse Isotope Dilution (LC-RID) method. The procedure involves the addition of 2.5 $\mu$g of non-radiolabeled STI571 to each sample as an internal standard. All blood samples are spiked with 100 $\mu$L of water for hemolysis whereas all other samples are directly analyzed. After adding 100 $\mu$L of concentrated buffer pH 12 and 4 mL of tert-butyl methylether, all samples are shaken for 15 min and centrifuged (6000 g, 10 min, 22°C). The organic layer is separated and evaporated in a vacuum centrifuge. The residues are dissolved in 250 $\mu$L of acetonitrile-water (30:70 v/v) followed by 75 $\mu$L of n-hexane. The reconstituted samples are then vortexed and centrifuged (13000 g, 4 min, 22°C), the top hexane layer is removed and 150$\mu$L are analyzed on HPLC. STI571 is separated from potential metabolites and endogenous compounds on an analytical Symmetry C-18 column, 3.5 $\mu$M, 150 x 46 mm (Waters, Milford, USA) equipped with a Symmetry C-18 pre-column, 5 $\mu$M, 20 x 3.9 mm (Waters, Milford, USA) at 10°C. The mobile phases A and B consists of water with 0.2 % formic acid, and acetonitrile with 0.2 % formic acid, respectively. The effluent is monitored by an UV-detector set at 270 nm. The retention time of STI571 peak is about 20 min. The peak corresponding to the unchanged compound is collected in a polyethylene vial using a fraction collector and analyzed for drug-related radioactivity by LSC. The concentration of [14C]STI571 in each sample is calculated.

The brain penetration of imatinib alone is low. Valspodar has no significant impact on the plasma pharmacokinetics of imatinib but strongly increases its brain penetration (brain-to-plasma concentration or AUC ratios x 5-6).

More specifically, after intravenous bolus of 12.5 mg/kg STI571:

- STI571 moderately distributes to tissues ($V_{dss}$ = 3.9 Ukg). The penetration of unchanged STI571 in brain is low, i.e. its brain-to-blood AUC ratio, Kp, amounts to 0.14.
- The brain exposure to STI571 -related metabolites is low, i.e. their brain-to-blood AUC ratio, Kp, amounts to 0.07.

After intravenous bolus of 12.5 mg/kg STI571 and 10 mg/kg PSC833:

- The disposition of STI571 -related metabolites is slightly modified. The blood exposure to metabolites is multiplied by a factor 1.3 in blood and 1.5 in plasma.
- The brain penetration of unchanged STI571 is highly enhanced by PSC833 (Tables 1 and 2), i.e. its brain-to-blood AUC ratio, Kp, amounts to 0.64. $C_{max}$ is still reached 15 minutes post dose but is multiplied by a factor 3.7, i.e. 4052 ng-eq/g in compound-related radioactivity or 3277 ng/g in unchanged STI571. Likewise, the brain exposure to unchanged STI571 is markedly enhanced (i.e. 5.4-fold increase) and the brain-to-blood concentration ratios for unchanged STI571 range from 0.55 to 0.95 (i.e. 4.8-fold increase on average). PSC833 does not significantly modify the elimination slope of unchanged STI571 from brain to blood ($t_{1/2z}$ = 2.4 h).

- After intravenous administration of 12.5 mg/kg [$^{14}$C]STI571 in rats, the fraction of unchanged STI571 among total radioactivity is more important in brain than in blood or in plasma, i.e. 80 % versus 65 % or 66 %, respectively. This demonstrates that the metabolites of STI571 penetrate weakly into the brain compartment as confirmed by the low metabolite concentrations in brain. The brain penetration of STI571 -related metabolites is highly increased by PSC833, i.e. their brain-to-blood AUC ratio, Kp, amounts to 0.31. Hence, brain exposure is multiplied by 6.8 and brain-to-blood Kp ratios (Table 3) by 4.4.

Example 4: Accumulation of CCP74588 into rat C6 glioma cells

Rat C6 glioma cells are used as a model for glioma in *in vitro* or *in vivo* studies, to investigate the effect of this combination on cell proliferation.

Rat C6 glioma cells are maintained in high glucose Dulbecco's modified medium supplemented with 10% fetal calf serum and 2 nM L-Glutamine in a humidified atmosphere of 95% air - 5% $CO_2$. All accumulation assays are conducted on cultures at confluence. For this purporse, the cells are seeded on 24-well plates at a density of $100 * 10^3$ cells/cm$^2$ and left to proliferate in culture medium for 48h. The accumulation of CGP74588 (0.5-50 µM) is measured after 0.5, 1, 2, 4, 8 or 16 min of incubation in a standard medium, which does not contain serum. For competition assays, the cultures are pre-incubated with various concentrations of the different competitors for 15 min. The accumulation of CGP74588 (1.5-50 µM) is measured after 1 min incubation in the presence of hydroxyurea (1, 10, 100 or 1000 µM), valspodar (1 or 10 µM), zosuquidar (1 or 10 µM), elacridar (1 or 10 µM), carbamazepine (10 or 100 µM) or phenytoin (10 or 100 µM). The cellular accumulation of 1.5 µM CGP74588 is also studied in C6 cells that have been pre-incubated with hydroxyurea (1, 10, 100 or 1000 µM) for 48 h.

The accumulation of CGP74588 in rat C6 cells is concentration dependant, suggesting that one or more saturable transport system influence its uptake in these cells. The accumulation of CGP74588 in rat C6 cells after 1 min incubation is significantly increased after 15 min preincubation of the cells with valspodar, zosuquidar and elacridar. All three inhibitors have a similar effect of CGP74588 accumulation at the same concentration (average 1.7-fold increase at 1 µM and average 2.3-fold increase at 10 µM).

**Table 1: Brain kinetics of radioactivity in group B**

Concentrations of compound-related radioactivity (ng-eq/g) in brain corrected from blood contamination (4.7 %) and pharmacokinetic parameters after intravenous administration of 12.5 mg/kg [$^{14}$C]STI571 and 10 mg/kg PSC833 to male Hanover Wistar rats (n=3 per time point).

| Time (h) | Individual data | | | Mean | SD | %CV |
|---|---|---|---|---|---|---|
| 0.25 | 4321 | 4767 | 3069 | 4052 | 881 | 22 |
| 0.5 | 3987 | 3205 | 4123 | 3772 | 495 | 13 |
| 1 | 2583 | 2504 | 3362 | 2816 | 474 | 17 |
| 2 | 1655 | 2716 | 2102 | 2158 | 533 | 25 |
| 4 | 850 | 875 | 924 | 883 | 38 | 4 |
| 8 | 378 | 429 | 294 | 367 | 68 | 19 |
| 24 | 75 | 92 | 104 | 91 | 15 | 16 |
| Tmax | | | | 0.25 | | |
| $C_{max}$ | | | | 4052 | | |
| $C_{max}$/dose | | | | 324 | | |
| AUC$_{last}$ | | | | 10795 | | |
| AUC | | | | 12192 | | |
| AUC/dose | | | | 975 | | |
| $t_{1/2z}$ | | | | 2.6 | | |
| AUC$_{brain/blood}$ | | | | 0.51 | | |

(continued)

Concentrations of compound-related radioactivity (ng-eq/g) in brain corrected from blood contamination (4.7 %) and pharmacokinetic parameters after intravenous administration of 12.5 mg/kg [14C]STI571 and 10 mg/kg PSC833 to male Hanover Wistar rats (n=3 per time point).

| Time (h) | Individual data | Mean | SD | %CV |
|---|---|---|---|---|
| $AUC_{brain/plasma}$ | | 0.39 | | |

Rsq > 0.97. LOQ = 27 ng-eq/g before vascular contamination correction.

$T_{max}$ in h, $C_{max}$ in ng/g, $C_{max}$/dose in (ng/g)/(mg/kg), $AUC_{last}$ and AUC in h ng-eq/g, AUC/dose in (h ng-eq/g)/(mg/kg), $t_{1/2z}$ in h, $AUC_{brain/blood}$ or plasma is the ratio of $AUC_{brain}$ versus $AUC_{blood}$ or plasma for compound-related radioactivity. To get pmol/g concentrations, multiply these ng-eq/g levels by factor 2.03.

**Table 2: Brain kinetics of STI571 in group A**

Concentrations of STI571 (ng/g) in brain corrected from blood contamination (4.7 %) and pharmacokinetic parameters after intravenous administration of 12.5 mg/kg [14C]STI571 to male Hanover Wistar rats (n=3 per time point).

| Time (h) | Individual data | | | Mean | SD | %CV |
|---|---|---|---|---|---|---|
| 0.25 | 746 | 494 | 1438 | 893 | 488 | 55 |
| 0.5 | 652 | 717 | 229 | 533 | 265 | 50 |
| 1 | 540 | 506 | 604 | 550 | 50 | 9 |
| 2 | 402 | 275 | 322 | 333 | 64 | 19 |
| 4 | 102 | 112 | 131 | 115 | 14 | 12 |
| 8 | 52 | 33 | 29 | 38 | 12 | 32 |
| 24 | < LOQ | < LOQ | < LOQ | - | - | - |
| $T_{max}$ | | | | 0.25 | | |
| $C_{max}$ | | | | 893 | | |
| $C_{max}$/dose | | | | 71 | | |
| $AUC_{last}$ | | | | 1678 | | |
| AUC | | | | 1802 | | |
| AUC/dose | | | | 144 | | |
| $t_{1/2z}$ | | | | 2.2 | | |
| $f_{unchanged}$ | | | | 79.9 | | |
| $AUC_{brain/blood}$ | | | | 0.14 | | |
| $AUC_{brain/plasma}$ | | | | 0.11 | | |

Rsq > 0.97. LOQ = 49 ng/g before vascular contamination correction.

$T_{max}$ in h, $C_{max}$ in ng/g, $C_{max}$/dose in (ng/g)/(mg/kg), $AUC_{last}$ and AUC in h ng/g, AUC/dose in (h ng/g)/(mg/kg), $t_{1/2z}$ in h. $f_{unchanged}$ = fraction unchanged (%) based on brain AUC parent drug/radioactivity ratio. $AUC_{brain/blood}$ or plasma is the ratio of $AUC_{brain}$ versus $AUC_{blood}$ or plasma for STI571. To get pmol/g concentrations, multiply these ng/g levels by factor 2.03.

**Table 3: Brain penetration of STI 571 -related metabolites in group B**

[A] Brain-to-blood concentration ratios for STI 571-related metabolites after intravenous administration of 12.5 mg/kg [14C]STI571 and 10 mg/kg PSC833 to male Hanover Wistar rats (n=3 per time point).

| Time (h) | Individual data | | | Mean | SD | %CV |
|---|---|---|---|---|---|---|
| 0.25 | 0.54 | 0.62 | 0.28 | 0.48 | 0.18 | 37 |
| 0.5 | 0.49 | 0.35 | 0.43 | 0.42 | 0.07 | 16 |
| 1 | 0.26 | 0.41 | 0.47 | 0.38 | 0.11 | 28 |
| 2 | 0.31 | 0.50 | 0.38 | 0.40 | 0.10 | 24 |
| 4 | 0.27 | 0.23 | 0.27 | 0.25 | 0.03 | 10 |

(continued)

[A] Brain-to-blood concentration ratios for STI 571-related metabolites after intravenous administration of 12.5 mg/kg [$^{14}$C]STI571 and 10 mg/kg PSC833 to male Hanover Wistar rats (n=3 per time point).

| Time (h) | Individual data | | | Mean | SD | %CV |
|---|---|---|---|---|---|---|
| 8 | 0.40 | 0.39 | 0.24 | 0.34 | 0.09 | 26 |
| 24 | nd | nd | Nd | - | - | - |

[B] Brain-to-plasma concentration ratios for STI571 -related metabolites after intravenous administration of 12.5 mg/kg [$^{14}$C]STI571 and 10 mg/kg PSC833 to male Hanover Wistar rats (n=3 per time point).

| Time (h) | Individual data | | | Mean | SD | %CV |
|---|---|---|---|---|---|---|
| 0.25 | 0.38 | 0.34 | 0.18 | 0.30 | 0.11 | 35 |
| 0.5 | 0.32 | 0.27 | 0.28 | 0.29 | 0.03 | 9 |
| 1 | 0.15 | 0.23 | 0.28 | 0.22 | 0.06 | 28 |
| 2 | 0.21 | 0.32 | 0.24 | 0.25 | 0.06 | 23 |
| 4 | 0.20 | 0.13 | 0.17 | 0.17 | 0.03 | 21 |
| 8 | 0.26 | 0.27 | 0.15 | 0.23 | 0.07 | 29 |
| 24 | nd | nd | Nd | - | - | - |

nd = non determined

**Claims**

1. Use of a combination comprising

   (a) N-{5-[4-(4-methyl-piperazino-methyl)-benzoylamido]-2-methylphenyl}-4-(3-pyridyl)-2-pyrimidine-amine as only active ingredient inhibiting the BCRP efflux pump,
   (b) at least one compound inhibiting an efflux pump selected from MDR1 and MRP2 selected from the group consisting of:

   (1) [3'-desoxy-3'-oxo-MeBmt]$^1$-Ciclosporin,
   (2) [3'-desoxy-3'-oxo-MeBmt]$^1$-[Val]$^2$-Ciclosporin,
   (3) [3'-desoxy-3'-oxo-MeBmt]$^1$-[Nva]$^2$-Ciclosporin,
   (4) Cyclo-[Pec-MeVal-Val-MeAsp(f3-P-t-Bu)-MeIle-MeIle-Gly-MeVal-Tyr(Me)-L-Lact],
   (5) Cyclo-[Pec-MeVal-Val-MeAsp-MeIle-MeIle-Gly-MeVal-Tyr(Me)-D-Lact], and
   (6) Verapamil,

   (c) optionally an anti-neoplastic agent,
   (d) optionally an alkylating agent, and
   (e) optionally hydroxyurea,

   wherein the active ingredients are present in each case in free form or in the form of a pharmaceutically acceptable salt or any hydrate thereof, and optionally at least one pharmaceutically acceptable carrier; for simultaneous, separate or sequential use for the preparation of a medicament for the treatment of a solid tumor disease.

2. The use according to claim 1 wherein N-{5-[4-(4-methyl-piperazino-methyl)-benzoylamido]-2-rnethylphenyl}-4-(3-pyridyl)-2-pyrimidine-amine is used in the form of its monomethanesulfonate salt.

3. The use according to any one of claims 1, 2, wherein the solid tumor disease is located in the central nervous system.

4. The use according to any one of claims 1, 2, wherein the solid tumor disease is glioma.

5. The use according to claim 3 or 4 wherein the combination comprises an alkylating agent, especially temozolomide.

6. The use according to any one of claims 3 to 5 wherein the combination comprises hydroxyurea.

7. The use according to any one of the preceding claims, wherein the combination comprises a compound inhibiting the MDR1 efflux pump selected from verapamil, [3'-desoxy-3'-oxo-MeBmt][1]-Ciclosporin, [3'-desoxy-3'-oxo-MeB-mt][1]-[Val][2]-Ciclosporin and [3'-desoxy-3'-oxo-MeBmt][1]-[Nva][2]-Ciclosporin.

8. The use according to any one of the preceding claims, wherein the combination comprises a compound inhibiting the MDR1 efflux pump, wherein said compound is valspodar (PSC833).

9. The use according to any one of preceding claims, wherein 25 to 1000 mg/day of N-{5-[4-(4-methyl-piperazino-methyl)-benzoylamido]-2-rnethylphenyl)-4-(3-pyridyl)-2-pyrimidine-amine monomesylate is administered to a human patient.

**Patentansprüche**

1. Verwendung einer Kombination, umfassend

   (a) N-{5-[4-(4-Methyl-piperazino-methyl)-benzoylamido]-2-methylphenyl)-4-(3-pyridyl)-2-pyrimidinamin als dem einzigen aktiven Inhaltsstoff, der die BCRP Effluxpumpe inhibiert,
   (b) mindestens eine Verbindung, die eine Effluxpumpe ausgewählt aus MDR1 und MRP2 inhibiert, ausgewählt aus der Gruppe bestehend aus:

   (1) [3'-Desoxy-3'-oxo-MeBmt][1]-Ciclosporin,
   (2) [3'-Desoxy-3'-oxo-MeBmt][1]-[Val][2]-Ciclosporin,
   (3) [3'-Desoxy-3'-oxo-MeBmt][1]-[Nva][2]-Ciclosporin,
   (4) Cyclo-[Pec-MeVal-Val-MeAsp($\beta$-P-t-Bu)-Melle-Melle-Gly-MeVal-Tyr(Me)-L-Lact],
   (5) Cyclo-[Pec-MeVal-Val-MeAsp-Melle-Melle-Gly-MeVal-Tyr(Me)-D-Lact], und
   (6) Verapamil,

   (c) gegebenenfalls einem anti-neoplastischen Mittel,
   (d) gegebenenfalls einem alkylierenden Mittel und
   (e) gegebenenfalls Hydroxyurea,

   wobei die aktiven Inhaltsstoffe in jedem Fall in freier Form oder in Form eines pharmazeutisch akzeptablen Salzes oder irgendeines Hydrats davon vorhanden sind, und gegebenenfalls mindestens einem pharmazeutisch akzeptablen Träger; zur gleichzeitigen, getrennten oder sequentiellen Verwendung zur Herstellung eines Medikaments zur Behandlung einer soliden Tumorerkrankung.

2. Verwendung nach Anspruch 1, wobei N-{5-[4-(4-Methyl-piperazino-methyl)-benzoylamido]-2-rnethylphenyl)-4-(3-pyridyl)-2-pyrimidinamin in Form seines Monomethansulfonatsalzes verwendet wird.

3. Verwendung nach einem der Ansprüche 1, 2, wobei die solide Tumorerkrankung im zentralen Nervensystem lokalisiert ist.

4. Verwendung nach einem der Ansprüche 1, 2, wobei die solide Tumorerkrankung ein Gliom ist.

5. Verwendung nach Anspruch 3 oder 4, wobei die Kombination ein alkylierendes Mittel umfasst, insbesondere Temozolomid.

6. Verwendung nach einem der Ansprüche 3 bis 5, wobei die Kombination Hydroxyurea umfasst.

7. Verwendung nach einem der voranstehenden Ansprüche, wobei die Kombination eine die MDR1 Effluxpumpe inhibierende Verbindung ausgewählt aus Verapamil, [3'-Desoxy-3'-oxo-MeBmt][1]-Ciclosporin, [3'-Desoxy-3'-oxo-MeBmt][1]-[Val][2]-Ciclosporin und [3'-Desoxy-3'-oxo-MeBmt][1]-[Nva][2]-Ciclosporin umfasst.

8. Verwendung nach einem der voranstehenden Ansprüche, wobei die Kombination eine die MDR1 Effluxpumpe inhibierende Verbindung umfasst, wobei die Verbindung Valspodar (PSC833) ist.

9. Verwendung nach einem der voranstehenden Ansprüche, wobei 25 bis 1000 mg/Tag an N-{5-[4-(4-Methyl-piperazino-methyl)-benzoylamido]-2-methylphenyl)-4-(3-pyridyl)-2-pyrimidinaminmonomesylat an einen menschlichen Patenten verabreicht werden.

**Revendications**

1. Utilisation d'une combinaison comprenant

(a) de la N-{5-[4-(4-méthyl-pipérazino-méthyl)-benzoyl-amido]-2-méthylphényl}-4-(3-pyridyl)-2-pyrimidine-amine comme seul principe actif inhibant la pompe à efflux de la BCRP,
(b) au moins un composé inhibant une pompe à efflux choisie parmi MDR1 et MRP2, choisi dans le groupe constitué pat :

(1) la [3'-désoxy-3'-oxo-MeBmt][1]-ciclosporine,
(2) la [3'-désoxy-3'-oxo-MeBmt][1]-[Val][2]-ciclosporine,
(3) la [3'-désoxy-3'-oxo-MeBmt][1]-[Nva][2]-ciclosporine,
(4) le cyclo-[Pec-MeVal-Val-MeAsp(β-P-t-Bu)Melle-Melle-Gly-MeVal-Tyr(Me)-L-Lact],
(5) le cyclo-[Pec-MeVal-Val-MeAsp-Melle-Melle-Gly-MeVal-Tyr(Me)-D-Lact], et
(6) le vérapamil,

(c) éventuellement un agent antinéoplasique,
(d) éventuellement un agent alkylant, et
(e) éventuellement une hydroxyurée,

où les principes actifs sont présents dans chaque cas sous forme libre ou sous la forme d'un sel pharmaceutiquement acceptable ou de tout hydrate de celui-ci, et éventuellement au moins un support pharmaceutiquement acceptable ; pour une utilisation simultanée, séparée ou séquentielle pour la préparation d'un médicament destiné au traitement d'une maladie de tumeur solide.

2. Utilisation selon la revendication 1, où la N-{5-[4-(4-méthyl-pipérazino-méthyl)benzoylamido]-2-méthylphényl}-4-(3-pyridyl)-2-pyrimidine-amine est utilisée sous la forme de son sel monométhanesulfonate.

3. Utilisation selon l'une quelconque des revendications 1, 2 où la maladie de tumeur solide est localisée dans le système nerveux central.

4. Utilisation selon l'une quelconque des revendications 1, 2, où la maladie de tumeur solide est un gliome.

5. Utilisation selon la revendication 3 ou 4, où la combinaison comprend un agent alkylant, spécialement le témozolomide.

6. Utilisation selon l'une quelconque des revendications 3 à 5, où la combinaison comprend une hydroxyurée.

7. Utilisation selon l'une quelconque des revendications précédentes, où la combinaison comprend un composé inhibant la pompe à efflux de MDR1, choisi parmi le vérapamil, la [3'-désoxy-3'-oxo-MeBmt]'-ciclosporine, la [3'-désoxy-3'-oxo-MeBmt][1]-[Val][2]-ciclosporine et la [3'-désoxy-3'-oxo-MeBmt][1]-[Nva][2]-ciclosporine.

8. Utilisation selon l'une quelconque des revendications précédentes, où la combinaison comprend un composé inhibant la pompe à efflux de MDR1, où ledit composé est le valspodar (PSC833).

9. Utilisation selon l'une quelconque des revendications précédentes, où 25 à 1 000 mg/jour de monomésylate de N-{5-[4-(4-méthyl-pipérazino-méthyl)benzoylamido]-2-méthylphényl}-4-(3-pyridyl)-2-pyrimidine-amine sont administrés à un patient humain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006012958 A
- US 5521184 A
- WO 9903854 A
- WO 03090720 A
- US 20040102453 A
- EP 0296122 A
- EP 0360760 A
- WO 9320833 A
- EP 0236940 A
- US 4978672 A
- JP 2018112 A
- US 4659516 A
- WO 9917804 A
- WO 9835958 A
- WO 0009495 A
- WO 0027820 A
- WO 0059509 A
- WO 9811223 A
- WO 0027819 A
- EP 0769947 A
- WO 0037502 A
- WO 9410202 A

- WO 9702266 A
- EP 0564409 A
- EP 0520722 A
- EP 0566226 A
- EP 0787722 A
- EP 0837063 A
- US 5747498 A
- WO 9810767 A
- WO 9730034 A
- WO 9749688 A
- WO 9738983 A
- WO 9633980 A
- EP 0296110 A
- WO 0048571 A
- US 4100274 A
- US 5843901 A
- US 4636505 A
- EP 0275821 A
- US 4939130 A
- JP 2744238 B
- US 0114886 A
- GB 2177700 A

**Non-patent literature cited in the description**

- **G. Dresemann.** *ASCO,* 2004
- **Reardon et al.** *J Clin Oncol.,* 20 December 2005, vol. 23 (36), 9359-68
- **Dresemann.** *Ann Oncol.,* October 2005, vol. 16 (10), 1702-8
- **Breedveld P ; Puim D ; Cipriani G ; Wielinga P ; van Tellingen O ; Schinkel AH et al.** The effect of Bcrpi (Abcg2) on the in vivo pharmacokinetics and brain penetration of imatinib mesylate (Gleevec): implications for the use of BCRP and P-gp inhibitors to enable the barin penetration of imatinib in patients. *Cancer Res.,* 01 April 2005, vol. 65 (7), 2577-82
- **Mahon, F-X. et al.** *Blood,* 2003, vol. 101 (6), 2368-2373

- **Dai H. et al.** *J. Pharm. Exp. Ther.,* 2003, vol. 304 (3), 1085-1092
- **M. Prewett et al.** *Cancer Research,* 1999, vol. 59, 5209-5218
- **F. Yuan et al.** *Proc. Natl. Acad. Sci. USA,* December 1996, vol. 93, 14765-14770
- **Z. Zhu et al.** *Cancer Res.,* 1998, vol. 58, 3209-3214
- **J. Mordenti et al.** *Toxicologic Pathology,* 1999, vol. 27 (1), 14-21
- **M. S. O'Reilly et al.** *Cell,* 1994, vol. 79, 315-328
- **M. S. O'Reilly et al.** *Cell,* 1997, vol. 88, 277-285